# FASCICULE DE BREVET EUROPEEN

(11) **EP 2 709 617 B1**
(45) Date de publication et mention de la délivrance du brevet: **25.11.2015**
(21) Numéro de dépôt: 12728638.3
(22) Date de dépôt: 14.05.2012
(51) Int. Cl.: A61K 31/4164, A61K 31/7052, A61K 9/00, A61K 31/00, A61P 11/02, A61K 45/06, A61K 31/7048

(54) **UTILISATION DU SECNIDAZOLE EN COMBINAISON DANS LE TRAITEMENT ORAL DES INFECTIONS DENTAIRES**
SECNIDAZOL ZUR VERWENDUNG IN KOMBINATION IN DER ORALEN BEHANDLUNG VON ZAHNINFEKTIONEN
SECNIDAZOLE FOR USE IN COMBINATION IN THE ORAL TREATMENT OF DENTAL INFECTIONS

(30) Priorité: 16.05.2011 FR 1101478
(43) Date de publication de la demande: 26.03.2014
(73) Titulaire: Vegefarm, 75010 Paris (FR)
(72) Inventeur: DEFRANCE, Pierre-Marie, F-13090 Aix-en-Provence (FR)
(74) Mandataire: Gicquel, Frédéric
(86) Numéro de dépôt international: PCT/FR2012/000192
(87) Numéro de publication internationale: WO 2012/156599

(56) Documents cités:
- ROTZETTER P A ET AL: "Kinetics of spiramycin/metronidazole (Rodogyl) in human gingival crevicular fluid, saliva and blood.", JOURNAL OF CLINICAL PERIODONTOLOGY OCT 1994 LNKD- PUBMED:7806675, vol. 21, no. 9, octobre 1994 (1994-10), pages 595-600, XP002658233, ISSN: 0303-6979 cité dans la demande
- GAD HEBA A ET AL: "Formulation and evaluation of secnidazole or doxycycline dento-oral gels.", DRUG DEVELOPMENT AND INDUSTRIAL PHARMACY DEC 2008 LNKD- PUBMED:18785044, vol. 34, no. 12, décembre 2008 (2008-12), pages 1356-1367, XP009151294, ISSN: 1520-5762 cité dans la demande
- GAD HEBA A ET AL: "Formulation and evaluation of PLA and PLGA in situ implants containing secnidazole and/or doxycycline for treatment of periodontitis.", AAPS PHARMSCITECH 2008 LNKD- PUBMED:18654864, vol. 9, no. 3, 2008, pages 878-884, XP002658235, ISSN: 1530-9932 cité dans la demande
- GILLIS J C ET AL: "Secnidazole - A review of its antimicrobial activity, pharmacokinetic properties and therapeutic use in the management of protozoal infections and bacterial vaginosis", DRUGS, ADIS INTERNATIONAL LTD, NZ, vol. 51, no. 4, 1 janvier 1996 (1996-01-01), pages 621-638, XP009133139, ISSN: 0012-6667 cité dans la demande
- SHARMA J B ET AL: "Comparative efficacy of two regimens in syndromic management of lower genital infections", ARCHIVES OF GYNECOLOGY AND OBSTETRICS, SPRINGER, BERLIN, DE, vol. 273, no. 4, 1 janvier 2006 (2006-01-01), pages 232-235, XP019341424, ISSN: 1432-0711, DOI: 10.1007/S00404-005-0071-5
- MALHOLTRA M ET AL: "Ciprofloxacin-tinidazol combination, fluconazole-azithromicin-secnida zole-kit and doxycycline-metronidazole combination therapy in syndromic management of pelvic inflammatory disease: a prospective randomized controlled trial", INDIAN JOURNAL OF MEDICAL SCIENCES, BOARD OF THE INDIAN JOURNAL OF MEDICAL SCIENCES TRUST, BOMBAY, IN, vol. 57, no. 12, 1 décembre 2003 (2003-12-01), pages 549-555, XP003001449, ISSN: 0019-5359

## Description

Utilisation du secnidazole dans le traitement des infections dentaires

La présente invention concerne le traitement des infections stomatologiques aiguës, chroniques ou récidivantes, telles que :
▪ abcès dentaires, phlegmons, cellulites périmaxillaires, péricoronarites ;
▪ gingivites, stomatites ;
▪ parodontites ;
▪ parotidites, sous-maxillites, etc.

Le traitement le plus courant utilisé actuellement associe un nitroimidazole, à savoir le métronidazole, et un antibiotique du groupe des macrolides, à savoir la spiramycine. J. Clin. Periodontol 1994 : 21 : 595-600 rapporte en effet le résultat de travaux menés sur l'association métronidazole / spiramycine de marque Rodogyl® pour le traitement de la parodontite. Selon ce document, le Rodogyl® utilisé, spécialement préparé, renferme 739 000 UI de spiramycine et 127,4 mg de métronidazole (page 596, colonne centrale, 2^{ème} paragraphe).

Il ressort en outre du même document que le traitement oral examiné dure 3 jours et comprend l'administration de 6 comprimés le 1^{er} jour, 6 comprimés le 2^{ème} jour et 3 comprimés le 3^{ème} jour (page 596, colonne de droite, avant dernier paragraphe), soit 15 comprimés, ce qui représente :
- 11 085 000 UI (= 3454 mg) de spiramycine (soit 4,10 fois la masse molaire de cet antibiotique macrolide), et
- 1911 mg de métronidazole.

Il serait souhaitable d'alléger ce traitement et c'est là le but que s'est fixé l'invention.

Le secnidazole, ou alpha-2-diméthyl-5-nitro-1*H-*imidazole-1-éthanol, est indiqué dans les cas d'amibiase, de trichomonase ou de giardiase (Jane C. Gillis et al, Data Embase [online] EMB-1996113854 et MLM Gonzalez et al, Data Embase [online] EMB-2009489088).

Bien que le secnidazole soit voisin, sur le plan de la structure, du métronidazole, il n'a pas été envisagé, à ce jour, de l'utiliser pour le traitement par voie orale des infections stomatologiques en association avec un antibiotique de la famille des macrolides.

Or, il est apparu à la déposante que le secnidazole pouvait être de nature à alléger le traitement de telles infections, compte tenu de sa longue demi-vie dans l'organisme et elle a constaté que, non seulement une prise unique de secnidazole, présente un rapport bénéfice-risque au moins équivalent au traitement de référence actuel mais, encore, et de manière tout à fait inattendue, que l'administration concomitante de l'antibiotique du groupe macrolide peut, elle aussi, être ramenée à 1 jour au lieu de 3-4 jours.

Par prise unique, on entend ici un seul jour de traitement, en une seule prise ou éventuellement en deux prises.

La prise unique renfermera généralement de 1500 mg à 2500 mg, et de préférence 2000 mg de secnidazole.

L'invention porte donc sur l'utilisation du secnidazole en association avec un antibiotique du groupe des macrolides, pour l'obtention d'un médicament unitaire ou d'un couple de médicaments destiné(s) au traitement par voie orale des infections stomatologiques.

Cet antibiotique peut être la spiramycine comme dans l'association, tombée dans le domaine public, exploitée sous la marque Rodogyl®, et dans laquelle personne, avant la déposante, n'a eu l'idée de substituer le secnidazole au métronidazole, en dépit du fait qu'il est connu depuis longtemps que la durée de vie plasmatique du secnidazole est supérieure à celle du métronidazole. La simple parenté de structure n'était donc pas une incitation suffisante pour l'homme du métier.

L'absence d'évidence de cette substitution est d'autant plus grande que l'on avait pourtant déjà envisagé d'utiliser le secnidazole dans le traitement d'une affection dentaire.

Ainsi, Drug Development and Industrial Pharmacy, 34 :1356-1367, 2008 - pages 1356 à 1367) évalue le secnidazole formulé en gel dento-oral pour une administration directement dans la poche parodontale.

Cependant, l'utilisation prévue selon ce document est
- non pas par voie orale (= systémique) comme proposé par la présente invention, mais par voie locale et qui plus est, par une méthode qui ne peut être mise en oeuvre que par un professionnel de la santé, non par le patient lui-même, puisqu'il s'agit d'injecter le gel avec une seringue dans la poche parodontale,
- non pas en associant le secnidazole avec un antibiotique de type macrolide, mais en l'employant seul,
- sans que la durée du traitement ou le nombre d'injections soit précisé, tous les tests rapportés étant des tests in vitro [la seule intervention sur des patients (colonne de gauche p. 1359) étant un prélèvement en vue d'une culture et d'un essai in vitro], et
- sans que la quantité de secnidazole nécessaire au traitement soit précisée : le document se borne à indiquer que la concentration inhibitrice minimale vis-à-vis des microorganismes testés est de 0,125-5 µg de secnidazole/ml de gel.

Cet article de Drug Development and Industrial Pharmacy qui ne décrit qu'un gel pour application locale et ne renfermant que du secnidazole, ne peut suggérer par référence implicite au traitement des vaginoses **(référence citée Gillis & Wiseman¹),** de substituer le secnidazole au métronidazole dans l'association à laquelle se rapporte J. Clin. Periodontol 1994 : 21 : 595-600 pour faire passer la durée du traitement de 3 jours à 1 jour, et réduire considérablement la quantité d'antibiotique.
¹ Gillis, J.C. & Wiseman, L.R. (1996) Secnidazole : A review of its antimicrobial activity, pharmacokinetic properties and therapeutic use in the management of protozoal infections and bacterial vaginosis. Drugs, 51, 621-638.

AAPS PharmaSciTech, Vol.9, No. 3, September 2008-pages 878 à 884, qui a pour auteurs les mêmes que l'article de Drug Development and Industrial Pharmacy analysé ci-dessus, en reprend une partie du texte, mot pour mot, la différence entre les deux étant toutefois qu'au lieu d'un gel administré à la seringue dans la poche parodontale, ils préconisent cette fois l'insertion d'un implant réalisé in situ dans ladite poche, implant qui associe le secnidazole et le chlorhydrate de doxycycline.

La doxycycline n'est pas un macrolide mais une tétracycline. L'antibiotique associé au secnidazole n'appartient donc pas à la même catégorie que ceux prévus par l'invention. Quant à la référence 12 citée dans AAPS PharmaSciTech, relative à la demi-vie du secnidazole, ce n'est autre que la référence Gillis & Wiseman citée dans l'article de Drug Development and Industrial Pharmacy : elle concerne le traitement des vaginoses.

En bref, à part l'idée d'associer un antibiotique au secnidazole, l'article de AAPS PharmaSciTech n'apporte rien de plus que l'article de Drug Development and Industrial Pharmacy, puisque l'utilisation prévue selon l'article de AAPS PharmaSciTech est
- non pas par voie orale (= systémique) comme proposé par la présente invention, mais par voie locale avec recours à une méthode qui ne peut être mise en oeuvre que par un professionnel de la santé, non par le patient lui-même puisqu'il s'agit de réaliser l'implant in situ dans la poche parodontale,
- non pas en associant le secnidazole avec un antibiotique de type macrolide, mais avec un antibiotique de type tétracycline,
- sans que la durée du traitement ou le nombre d'implantations soit précisé, tous les tests rapportés étant des tests in vitro et la seule intervention sur des patients (colonne de gauche p. 880) étant un prélèvement en vue d'une culture et d'un essai in vitro (l'article de AAPS PharmaSciTech ne prétend d'ailleurs pas que des résultats in vivo aient été obtenus : il se borne à indiquer, page 883, colonne de droite, fin du 3^{ème} paragraphe, que l'activité est *prometteuse),* et
- sans que la quantité de secnidazole et d'antibiotique nécessaire au traitement soit précisée.

Associée au secnidazole, dans l'application thérapeutique et les conditions d'administration prévue (une seule prise), la spiramycine sera employée à une dose de l'ordre de 3 000 000 UI.

De préférence, toutefois, comme antibiotique macrolide, il sera associé au secnidazole l'azithromycine, à une dose comprise entre 750 et 1500 mg, de préférence 1000 mg.

Si l'on compare avec les chiffres indiqués plus haut pour le Rodogyl®, le traitement oral proposé par l'invention consiste en l'administration d'une prise unique (= 1 seul jour), de préférence, de
- 1000 mg d'azithromycine (soit 1,34 fois la masse molaire de cet antibiotique macrolide), et
- 2000 mg de secnidazole

Comme on le voit, une quantité de secnidazole sensiblement voisine de la quantité de métronidazole permet non seulement de réduire de 3 jours à 1 jour la durée du traitement, mais aussi de diviser par 3 la quantité d'antibiotique administrée.

Selon l'invention, le traitement peut donc se résumer en un jour (au lieu des 3-4 jours) où le patient recevra, par voie orale, la dose précitée de secnidazole et soit une dose unique d'environ 1000 mg d'azithromycine, soit, de préférence, deux doses d'environ 500 mg d'azithromycine.

Le secnidazole et la dose unique (ou la première dose d'azithromycine) seront administrés simultanément ou en des temps rapprochés, et l'éventuelle seconde dose d'azithromycine 12 heures après la première prise.

De préférence, le secnidazole est sous forme pulvérulente et conditionné en sachet. L'azithromycine peut, de son côté, être également sous forme pulvérulente et conditionnée en sachet, ou se présenter sous la forme de comprimés.

En cas d'administration de l'antibiotique sous la forme d'une dose unique quotidienne, l'idéal sera que les deux principes actifs soient réunis dans une même forme pharmaceutique orale, auquel cas la poudre de secnidazole et la poudre d'azithromycine seront mélangées et conditionnées dans un même sachet, et le traitement consistera en l'ingestion du contenu de ce seul sachet.

Pour la mise en oeuvre de l'invention, il peut être proposé un kit comprenant, dans un conditionnement :
➢ une forme pharmaceutique adaptée à l'administration orale et renfermant de 1500 mg à 2500 mg, de préférence 2000 mg, de secnidazole, et de 750 mg à 1500 mg, de préférence 1000 mg, d'azithromycine et un excipient ou diluant pharmaceutiquement acceptable, ou
➢ d'une part, une forme pharmaceutique adaptée à l'administration orale et renfermant de 1500 mg à 2500 mg, de préférence 2000 mg, de secnidazole, et un excipient ou diluant pharmaceutiquement acceptable et, d'autre part, soit un exemplaire d'une forme pharmaceutique, adaptée à l'administration orale et renfermant 1000 mg d'azithromycine et un excipient ou diluant pharmaceutiquement acceptable, soit deux exemplaires d'une forme pharmaceutique, adaptée à l'administration orale et renfermant chacune 500 mg d'azithromycine et un excipient ou diluant pharmaceutiquement acceptable.

Il va de soi qu'un traitement s'effectuant sur un seul jour a beaucoup plus de chances d'être convenablement suivi, donc d'être actif, que le traitement actuel sur 3-4 jours.

De plus, un traitement raccourci, utilisant des antibiotiques de type macrolide, crée moins de germes mutants résistants. Enfin, on peut penser qu'un traitement plus court génère moins d'effets indésirables.

L'association selon l'invention a été testée dans le contexte suivant :
Les troisièmes molaires peuvent présenter au cours de leur éruption des manifestations inflammatoires locales appelées péricoronarites. Dans les formes aiguës, des signes régionaux et généraux peuvent y être associés (fièvre, adénopathies, trismus) ainsi que des complications éventuelles de type cellulite par diffusion de l'infection dans les espaces anatomiques de voisinage. Une antibiothérapie est alors justifiée, associée ou non à un geste chirurgical et, comme indiqué plus haut, l'association spiramycine-métronidazole (Rodogyl® ou sa variante doublement dosée Birodogyl®) est jusqu'ici principalement utilisée à cette fin.

DB, dentiste, face à des patients ayant des impératifs de guérison très rapide, a prescrit, au lieu du Birodogyl®, sur suggestion de la déposante et sous le sceau de la confidentialité, 2 g de secnidazole et 1 g d'azithromycine sur une seule journée. Il a constaté, dès le 3^{ème} jour après ce traitement, la disparition de l'oedème, de la suppuration et de la douleur, alors que, compte tenu de la demi-vie du secnidazole et de l'azithromycine, respectivement d'environ 25 heures et d'environ 20 heures, la durée d'efficacité du traitement antibiotique n'avait pas dépassé 25 heures, au lieu des 96 heures nécessaires dans le cas du Birodogyl®.

Il a recommencé l'opération chez une dizaine de patients chez qui il a constaté les mêmes résultats.

Cet effet est inattendu car il n'est pas directement lié à la durée de demi-vies des produits entrant dans la composition de l'association.

Il est bien entendu que l'invention n'est pas limitée à l'utilisation de la spiramycine et de l'azithromycine comme antibiotique, le secnidazole pouvant être associé à tout antibiotique convenable, actif contre les germes responsables des infections stomatologiques.

## Revendications

1. Composition pour une utilisation dans le traitement par voie orale des infections dentaires, comprenant du secnidazole en association avec un antibiotique de la famille des macrolides, le secnidazole et l'antibiotique étant associés au sein d'un médicament unitaire ou présentés sous la forme d'un couple de médicaments.

2. Composition pour une utilisation selon la revendication 1, **caractérisée en ce que** l'antibiotique est choisi entre la spiramycine et l'azithromycine.

3. Composition pour une utilisation selon la revendication 1 ou 2, **caractérisée en ce qu'**elle est conditionnée sous la forme d'un médicament unitaire ou de deux médicaments, comprenant,
➢ dans le premier cas, une forme pharmaceutique pour administration orale, renfermant de 1500 mg à 2500 mg, de préférence 2000 mg, de secnidazole, et de 750 mg à 1500 mg, de préférence 1000 mg d'azithromycine, et un excipient ou diluant pharmaceutiquement acceptable, ou
➢ dans le second cas, d'une part, une forme pharmaceutique pour administration orale, renfermant de 1500 mg à 2500 mg, de préférence 2000 mg, de secnidazole, et un excipient ou diluant pharmaceutiquement acceptable et, d'autre part, soit un exemplaire d'une forme pharmaceutique, adaptée à l'administration orale et renfermant 1000 mg d'azithromycine et un excipient ou diluant pharmaceutiquement acceptable, soit deux exemplaires d'une forme pharmaceutique, pour administration orale, renfermant chacune 500 mg d'azithromycine et un excipient ou diluant pharmaceutiquement acceptable.

## Patentansprüche

1. Zusammensetzung für eine Verwendung bei der Behandlung auf oralem Weg von Zahninfektionen, umfassend Secnidazol in Verbindung mit einem Antibiotikum der Familie der Makrolide, wobei das Secnidazol und das Antibiotikum innerhalb eines Einheitsarzneimittels assoziiert sind oder die Form eines Arzneimittelpaars aufweisen

2. Zusammensetzung für eine Verwendung nach Anspruch 1, **dadurch gekennzeichnet, dass** das Antibiotikum aus Spiramycin und Azithromycin ausgewählt ist.

3. Zusammensetzung für eine Verwendung nach Anspruch 1 oder 2, **dadurch gekennzeichnet, dass** sie in Form eines Einheitsarzneimittels oder von zwei Arzneimitteln hergestellt ist, umfassend:
- im ersten Fall eine pharmazeutische Form zur oralen Verabreichung, die von 1500 mg bis 2500 mg, vorzugsweise 2000 mg Secnidazol enthält, und von 750 mg bis 1500 mg, vorzugsweise 1000 mg Azithromycin, und ein pharmazeutisch verträgliches Bindemittel oder Verdünnungsmittel, oder
- im zweiten Fall einerseits eine pharmazeutische Form zur oralen Verabreichung, die von 1500 mg bis 2500 mg, vorzugsweise 2000 mg Secnidazol enthält, und ein pharmazeutisch verträgliches Bindemittel oder Verdünnungsmittel und andererseits entweder ein Exemplar einer pharmazeutischen Form, die für die orale Verabreichung ausgelegt ist und 1000 mg Azithromycin und ein pharmazeutisch verträgliches Bindemittel oder Verdünnungsmittel enthält, oder zwei Exemplare einer pharmazeutischen Form zur oralen Verabreichung, die jeweils 500 mg Azithromycin und ein pharmazeutisch verträgliches Bindemittel oder Verdünnungsmittel umfassen.

## Claims

1. Composition for use in the treatment *per os* of dental infections, comprising secnidazole in association with an antibiotic of the macrolide family, secnidazole and the antibiotic being associated in a unitary drug or presented in the form of a pair of drugs.

2. Composition for use according to claim 1, **characterised in that** the antibiotic is chosen between spiromycin and azithromycin.

3. Composition for use according to claim 1 or 2, **characterised in that** it is packaged in the form of a unitary drug or two drugs, comprising,
- in the first case, a pharmaceutical form for oral administration, containing 1500 mg to 2500 mg, preferably 2000 mg, of secnidazole, and 750 mg to 1500 mg, preferably 1000 mg of azithromycin, and a pharmaceutically acceptable excipient or diluent, or
- in the second case, firstly, a pharmaceutical form for oral administration, containing 1500 mg to 2500 mg, preferably 2000 mg, of secnidazole, and a pharmaceutically acceptable excipient or diluent and, secondly, either one unit of a pharmaceutical form, suitable for oral administration and containing 1000 mg of azithromycin and a pharmaceutically acceptable excipient or diluent, or two units of a pharmaceutical form, for oral administration, each containing 500 mg of azithromycin and a pharmaceutically acceptable excipient or diluent.
